(19) **Europäisches Patentamt / European Patent Office / Office européen des brevets**

(11) **EP 3 644 053 A1**

(12) **EUROPEAN PATENT APPLICATION**
published in accordance with Art. 153(4) EPC

(43) Date of publication:
**29.04.2020 Bulletin 2020/18**

(21) Application number: **18821393.8**

(22) Date of filing: **16.01.2018**

(51) Int Cl.:
**G01N 27/62** (2006.01)          **G08B 25/00** (2006.01)
**G08B 25/04** (2006.01)

(86) International application number:
**PCT/JP2018/000908**

(87) International publication number:
**WO 2018/235320 (27.12.2018 Gazette 2018/52)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD TN**

(30) Priority: **21.06.2017 JP 2017121580**

(71) Applicant: **Hitachi, Ltd.**
**Tokyo 100-8280 (JP)**

(72) Inventors:
- **KUMANO Shun**
  **Tokyo 100-8280 (JP)**
- **HASHIMOTO Yuichiro**
  **Tokyo 100-8280 (JP)**
- **SUGIYAMA Masuyuki**
  **Tokyo 100-8280 (JP)**
- **TAKADA Yasuaki**
  **Tokyo 100-8280 (JP)**

(74) Representative: **Strehl Schübel-Hopf & Partner**
**Maximilianstrasse 54**
**80538 München (DE)**

(54) **ADHERED MATTER INSPECTION METHOD**

(57)     To achieve inspection with both a high security level and high throughput, the present invention comprises: a step of acquiring personal information of a subject; a step of calculating a terrorism risk of the subject by referring the acquired personal information to a database storing a relationship between the personal information and the terrorism risk; and a step of using an inspection device for collecting an adhered matter on the subject or on an inspection object carried by the subject and/or a vapor from the adhered matter, ionizing and analyzing the collected matter, and determining whether or not the adhered matter is a dangerous material by referring the analysis result to the database; wherein inspection condition and/or judgement condition of the inspection device is changed on a per-subject basis so that a true positive rate of an inspection is varied according to a level of the calculated terrorism risk.

FIG. 6

EP 3 644 053 A1

## Description

Technical Field

The present invention relates to an adhered matter inspection method for inspecting an adhered matter on an inspection object or a subject or vapor from the adhered matter.

Background Art

Terrorist attacks have been committed around the world and the number of the attacks is over 10,000 per year. Death toll from terrorist attacks has been rising in spite of enhanced security measures and exceeded 30, 000 in 2014. Explosives have been used in half of the terrorist attacks, leading to an increasing demand for high-accuracy explosive detection devices in order to reduce the death toll. Particularly, heavy security is needed in airports because a few hundred grams of explosives can bring down an airplane. At present, explosive inspections conducted at airports include bulk detection and trace detection. The bulk detection uses an X-ray imaging device and a millimeter wave imaging device for determining the presence of some explosive on the basis of a configuration of the explosive. In the trace detection, on the other hand, an airport staff wipes off carry-on baggage so as to detect a trace of explosives by analyzing adhered trace component. It is necessary to take sufficient time to run a check in the interest of passenger safety. On the other hand, the number of airline passengers has been increasing every year. The airport cannot cope with all the increasing airline passengers unless time from check-in to boarding is shortened. A check time for security checkpoints constitutes a limiting factor of the above time problem. The improvement in inspection throughput is an urgent issue. Many of the airport passengers complain about such a long inspection time. Although such a lengthy inspection is for the sake of passenger safety, the low inspection throughput leads to low level satisfaction of the passengers. Just as described, a current issue is to achieve both high security level and high inspection throughput.

At airports, it is a common practice to change the security level in conformity to social conditions and the like. For example, in a case where terrorist attacks occur in various places, where a national event is held, or the like, the security level is raised by increasing the number of security guards and the number of security checkpoints and taking longer than usual to perform the inspection. However, the decrease in throughput is inevitable so that the satisfaction level of the passengers is lowered as described above.

A concept of "Risk based security" is disclosed in Non-Patent Literature 1. In the established view, all subjects passing through the security checkpoints are subjected to the inspection based on unified standards. However, the potential of the subject to commit the terrorist attack (hereinafter, referred to as "terrorism risk") varies greatly from person to person. The subjects are classified according to the terrorism risk and a subject with high terrorism risk is carefully checked over time while a subject with low terrorism risk is subjected to a simple inspection at high throughput. For instance, permission of carry-on electronic device or of carry-on liquid in container is changed depending upon the terrorism risk. The "Risk based security" is an attempt to optimize the inspection on the basis of each terrorism risk and to increase the inspection throughput without lowering the security level.

A method of increasing the inspection throughput by dividing the subjects into groups followed by collectively inspecting the subjects is disclosed in Patent Literature 1. There are provided at least two inspection devices, one of which is capable of simultaneously inspecting more than one subject. In a case where four subjects in a group are collectively checked by a first inspection device which gives a positive result in a certain group, for example, the four subjects in question are each put into each different group so as to be grouped with subjects determined to be negative in the first inspection. Then, the subjects in question are checked by a second inspection device. A positive subject can be identified based on the determination made by the second inspection device. This method is adapted to increase the inspection throughput by collectively checking the subjects on a per group basis. Further, the method is adapted to identify the positive subject by using a plurality of inspection devices in a step-by-step manner.

A method where an inspection route is changed in conformity to the conditions in view of the device accuracy and the inspection throughput is disclosed in Patent Literature 2. In this method, plural types of inspection routes using plural inspection devices in combination are previously defined. Considering the accuracies of individual devices and the inspection throughputs, the accuracy of each inspection route as a whole and the inspection throughput thereof are previously comprehended. In the case of high terrorism risk because of bad social situations, an inspection route giving priority to the inspection accuracy is selected in spite of low inspection throughput. In a case where the social conditions are stable and the increase in the number of subjects due to summer vacation or the like is prospected, an inspection route giving priority to the inspection throughput is selected. As just described, the method is directed to achieve the high inspection throughput and the high security level by selecting an optimum inspection route according to the conditions.

Citation List

Patent Literature

Patent Literature 1: Japanese Patent Application Laid-Open No. 2011-174746
Patent Literature 2: Japanese Patent Application Laid-Open No. 2009-80730

Non-Patent Literature

Non-Patent Literature 1: Journal of Air Transport Management, Vol.48, pp.60-64(2015)

Summary of Invention

Technical Problem

Nowadays when the terrorist attacks occur around the world, there is a demand for raising the security levels at places where large numbers of people gather, particularly at public transportation facilities such as airports. On the other hand, the number of passengers has been increasing every year and hence, the throughput of security inspection cannot be lowered. That is, there is a demand for performing the inspection with high throughput without lowering the security level.

Solution to Problem

According to an aspect of the invention, an adhered matter inspection method includes: a step of acquiring personal information of a subject; a step of calculating a terrorism risk of the subject by referring the acquired personal information to a database storing a relationship between the personal information and the terrorism risk; and a step of using an inspection device for collecting an adhered matter on the subject or on an inspection object carried by the subject and/or a vapor from the adhered matter, ionizing and analyzing the collected matter, and determining whether or not the adhered matter is a dangerous material by referring the analysis result to the database; and has a configuration wherein inspection condition and/or judgement condition of the inspection device is changed on a per-subject basis so that a true positive rate of an inspection is varied according to a level of the calculated terrorism risk.

Advantageous Effects of Invention

According the invention, the adhered matter can be inspected at high throughput without lowering the security level.
These and other objects, features and effects of the invention will become apparent from the following description of embodiments thereof.

Brief Description of Drawings

Fig. 1 is a conceptual diagram showing an adhered matter inspection system according to a first embodiment hereof;
Fig. 2 is a diagram illustrating the calculation of terrorism risk and algorithms for changing inspection condition and judgement condition;
Fig. 3 is a diagram showing an exemplary relation between state quantity and terrorism risk registered in a database;
Fig. 4 is a schematic diagram showing an exemplary configuration of an adhered matter inspection device;
Fig. 5 is a schematic diagram showing an exemplary configuration of the adhered matter inspection device;
Fig. 6 is a flow chart showing an exemplary inspection procedure;
Fig. 7 is a graph showing a typical example of the judgement condition changed on the basis of the terrorism risk;
Fig. 8 is a graph showing an exemplary change in the judgement condition;
Fig. 9 is a graph showing an exemplary change in the judgement condition;
Fig. 10 is a graph showing an exemplary mass spectrum;
Fig. 11 is a graph showing an exemplary tandem mass spectrometry; and
Fig. 12 is a set of graphs showing spectrum obtained by an ion mobility spectrometer and time-varying change in TNT signal.

Description of Embodiments

Next, a mode of carrying out the invention (hereinafter, referred to as "embodiment") is specifically described with reference to the accompanying drawings as needed.

While the accompanying drawings illustrate the specific embodiments of the invention in conformity with the principle of the invention, these are used for understanding of the invention but not for restrictive interpretation thereof. The invention includes the following embodiments and modifications made by combination with the known arts or replacement. Throughout the figures illustrating the embodiments, identical or equivalent elements will be referred to by like reference numerals and will be explained only once.

First Embodiment

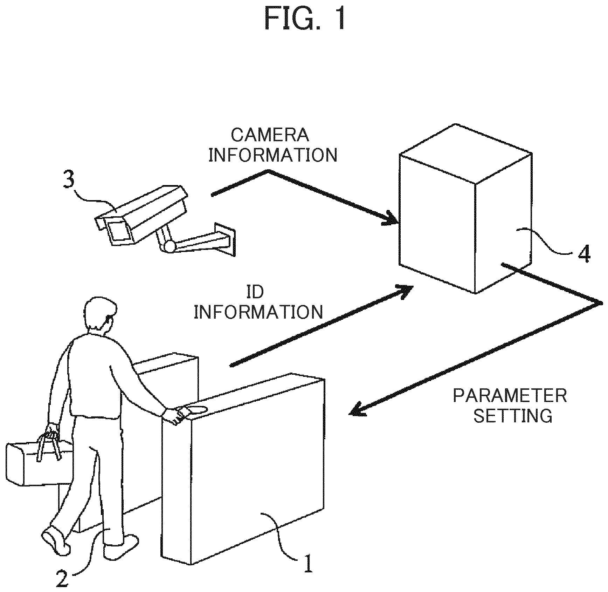

Now, a first embodiment of the invention is described with reference to Fig. 1 to Fig. 7.

Fig. 1 is a conceptual diagram of an adhered matter inspection system according to the first embodiment hereof. This adhered matter inspection system includes: a gate-type adhered matter inspection device 1 equipped with both an ID authentication function and a dangerous material detection function; a monitoring camera 3 and a server 4. A concept of an adhered matter inspection method according to the embodiment is described with reference to Fig. 1.

It is assumed that the system of the embodiment is used at a security checkpoint in an airport or the like. When a subject 2 approaches the gate-type adhered matter inspection device 1, the monitoring camera 3 acquires imagery information of the subject and transfers the acquired imagery information to the server 4. When passing through the gate-type adhered matter inspection device 1, the subject 2 need to go through ID authentication by means of the ID authentication function added to the adhered matter inspection device. The subject is identified by way of fingerprint, finger vein, iris, passport, two-dimensional barcode, smart phone or the like. ID information used for identifying the subject and acquiring personal information of the subject is transferred to the server 4. In parallel with the ID authentication, the gate-type adhered matter inspection device 1 determines whether an adhered matter on the subject 2 or on personal items thereof is a dangerous material or not. If the result of ID authentication and the result of adhered matter inspection are normal, the gate is opened to allow the passage of the subject.

It has been a common practice to carry out the security inspection on all the subjects 2 on the basis of unified criteria. However, the embodiment takes a different approach. The embodiment uses monitoring camera information and ID information to analyze the terrorism risk of the subject 2 on a real-time basis. The embodiment achieves both high security level and high throughput by changing the inspection condition and/or the judgement condition of the adhered matter inspection on a per-subject basis depending upon the level of terrorism risk. The monitoring camera 3 estimates the terrorism risk of the subject 2 from appearance information thereof. For example, the monitoring camera determines that a subject exhibiting an abnormal behavior has high risk.

The term "abnormal behavior" used herein means deviation from normal behaviors. The gate-type adhered matter inspection device is previously made to learn by machine learning normal behaviors of persons passing through the gate-type adhered matter inspection device so that the inspection device can determine a person deviated from the learned behavior patterns as a high-risk person. As will be described hereinlater, the gate-type adhered matter inspection device separates an adhered matter from a subject per se or from an inspection object carried by the subject by means of gas jet. Such a behavior as to block the gas jet is determined as a high-risk behavior. Further, such a behavior as to clean the inspection object, such as ID card or hand, with a handkerchief or the like just before the passage through the gate-type adhered matter inspection device is also determined as the high-risk behavior. This is because these behaviors are to prevent the inspection device from giving a positive result. Further, a behavior of deviating from a normal flow of people walking in vicinity of the gate-type adhered matter inspection device is also determined as the high-risk behavior. For example, such a behavior as to move against the lines of people walking toward the gate-type adhered matter inspection devices is determined as the high-risk behavior.

It is also possible to evaluate a tension state of the subject 2 on the basis of eye movement or slight body movement of the subject 2 and to use the evaluation result for calculating the terrorism risk. It is also possible to observe febrile state of the subject 2 by means of an infrared camera and to use the observation result for the calculation of the terrorism risk.

The size of a baggage such as a bag carried by the subject also affects the terrorism risk. The reason is because if a bomb is in the bag, the bigger the bomb, the heavier is the damage. In a case where the bag, such as a handbag or tote bag, is of a size as not to accommodate a big baggage, the subject has a low risk. In the case of a bag, such as a suitcase, which has such a size as to permit the carriage of a baggage as heavy as tens kilograms, the subject has a high risk.

The personal information of the subject as acquired through the ID authentication is also used for the calculation of the terrorism risk. A criminal record, for example, raises the terrorism risk. A record of overseas travel, the number of overseas travels, overseas travel destination and the like are also useful personal information. Further, a destination of overseas travel when the inspection is performed is quite helpful in the calculation of terrorism risk.

The monitoring camera information and ID information are transferred to the server 4, where the terrorism risk is calculated. The inspection condition or the judgement condition is calculated on the basis of the terrorism risk. The calculation result is transferred to the gate-type adhered matter inspection device 1 and applied to the inspection of the subject 2.

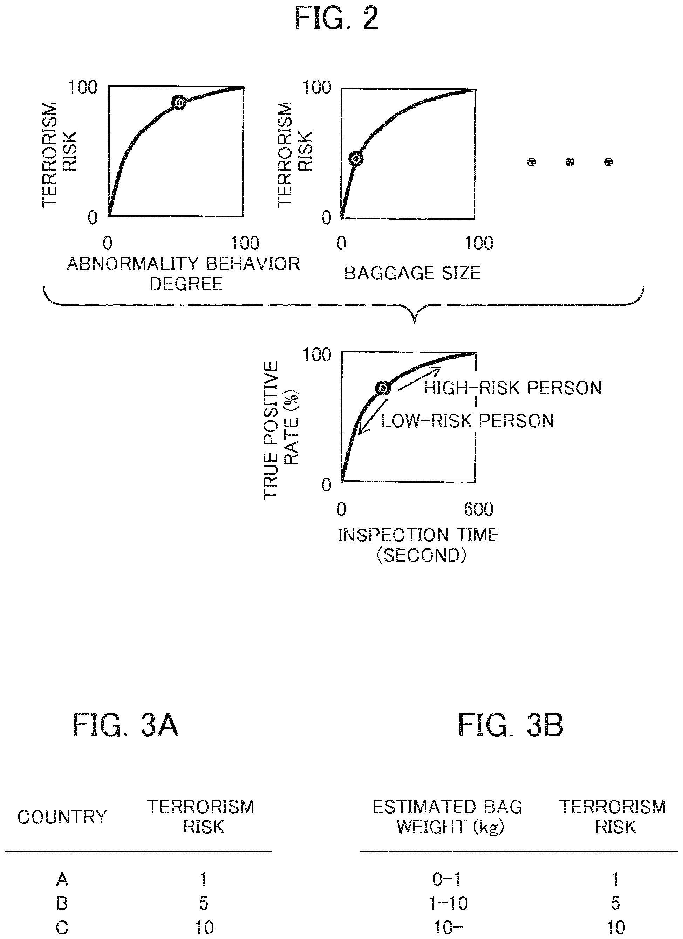

Fig. 2 is a diagram illustrating the calculation of terrorism risk and algorithms for changing the inspection condition and the judgement condition. As has been described above, the terrorism risk calculation does not use information on one subject only but uses information on a plurality of subjects. The server 4 stores a relation between the subject information acquired from the monitoring camera 3 and the ID information, and the terrorism risk. As illustrated by the graphs of Fig. 2, a relation between the degree of abnormal behavior and the terrorism risk, and a relation between the baggage size and the terrorism risk are stored. These relations do not always represent positive correlations like those shown in the graphs. The terrorism risk of a subject is calculated by using a plurality of subject information pieces. The calculation result is used for changing the inspection condition and/or the judgement condition.

As shown in Fig. 2, a relation between true positive rate and inspection time defines a curve representing the performance of the adhered matter inspection device. The true positive rate means the probability of an adhered matter on the subject being determined to be a dangerous material in a case where the adhered matter is the dangerous material. The inspection time means time taken to pass through the security checkpoint. In a case where the gate-type adhered matter inspection device 1 gives a positive result, for example, a re-inspection or detailed inspection is performed. The inspection time including such re-inspection time and/or detailed inspection time is plotted on the abscissa. Namely, the relation between the true positive rate and the inspection time as shown in Fig. 2 includes a concept of ROC (receiver operating characteristic) curve representing a relation between false positive rate and the true positive rate, the relation representing the performance of the device. In the case of the inspection device, if such a judgment condition as to raise the true positive rate is defined, the false positive rate rises, too. In a case where the positive result is given even though the result is false positive in nature, as described above, the inspection time is increased because the re-inspection or detailed inspection is performed. Therefore, the judgment condition so changed as to raise the true positive rate and the false positive rate means that conditions at an upper right portion of the characteristic curve of the graph showing the relation between the true positive rate and the inspection time is applied.

To put it simply, the embodiment is configured to calculate the terrorism risk of the subject so as to change the true positive rate of the device based on the calculation result. In a case where the positive rate for the subject with high terrorism risk is raised, the inspection time may be increased. Alternatively, such a judgement condition as to increase both the true positive rate and the false positive rate may be applied, resulting in the increased inspection time. At the security checkpoint applying the embodiment, the subject with low terrorism risk is subjected to the security inspection under the conditions of the low true positive rate and the short inspection time. The subject with high terrorism risk is subjected to the security inspection under the conditions of the high true positive rate and the long inspection time.

As described above, the terrorism risk of the subject is calculated on the basis of different information pieces on the subject. It is assumed that a relation between a state quantity $x_n$ of one information piece on the subject and a terrorism risk $y_n$ thereof is expressed as $y_n = f_n(X_n)$, where n takes an integer 1,2,3... for each information piece on the subject. If a bag size means an information piece on the subject, for example, x denotes an estimated weight of the bag. The state quantity $x_n$ does not always denote a numerical value but may sometimes denote country name or the like. In this case, a relation of x with y is as follows. If x denotes Japan, y takes a value 1, while if x denotes USA, y takes a value 2. In this form, the information is registered in a database. Fig. 3 is a diagram showing an exemplary relation between the state quantity and the terrorism risk registered in the database. The finally calculated terrorism risk y of the subject is calculated using the following expression.

$$ y = f_1(X_1) + f_2(X_2) + f_3(X_3)... \qquad (1) $$

Using the calculated terrorism risk y, a true positive rate r at the time of the inspection is calculated using an expression r = g(y). The inspection is performed under such measurement condition and judgement condition as to provide a true positive rate 'r' . For instance, assumed is a case where only an estimated bag weight is information usable in the calculation of the terrorism risk of the subject. If a bag weight estimated from a monitoring camera image is 30kg, then x = 30 which can be written, for example, as terrorism risk y = f (30) = 100. At this time, a true positive rate r = g(100) = 99 is given, for example. The inspection is performed under such measurement condition and judgement condition as to provide a true positive rate of 99%. The security level can be controlled by changing the relation of r = g(y) according to the social conditions.

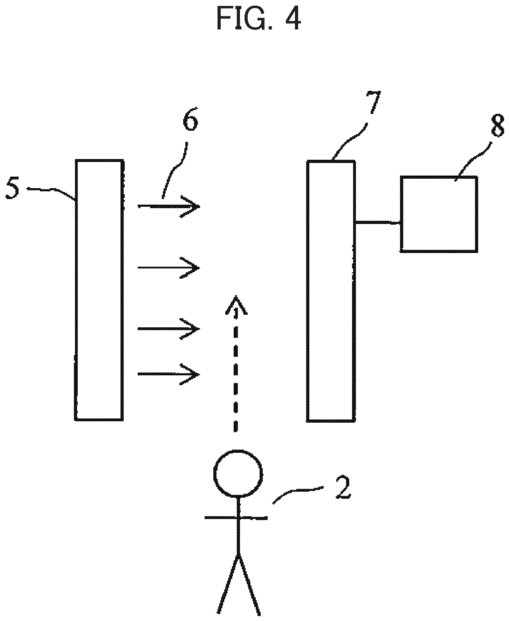

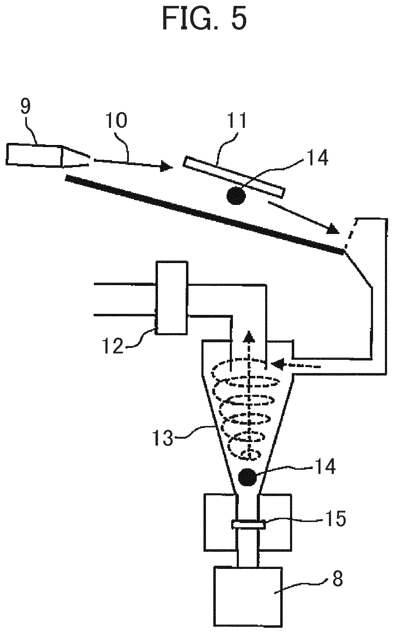

Fig. 4 and Fig. 5 are schematic diagrams showing exemplary configurations of the gate-type adhered matter inspection device 1 according to the first embodiment. The example of the adhered matter inspection device shown in Fig. 4 includes: a main machine 7, an auxiliary machine 5 and an ion analyzer 8 connected to the main machine 7. The subject 2 passes between the main machine 7 and the auxiliary machine 5. A hot air 6 is ejected from the auxiliary machine 5 such that the vaporization of the adhered matter on the subject 2 or on the personal items or clothes of the subject is promoted while the vaporized gas is collected by the main machine 7. The collected gas is ionized and analyzed by the ion analyzer 8. The gate-type adhered matter inspection device 1 is also equipped with an ID authentication function, which is not shown in the figure.

The adhered matter inspection device shown in Fig. 5 is an example of a device which analyzes an adhered matter 14 on an ID card as an inspection object 11 or on a hand holding the ID card, or on a lower sleeve edge of the subject's clothes or the like when the ID card is passed over the device for ID authentication. When the ID card is passed over the device, a gas 10 is ejected from a jet nozzle 9 so as to separate the adhered matter 14 from the ID card. The adhered matter 14 so separated is sucked by a suction fan 12 and introduced into a condenser such as a cyclone 13 for condensation. A heated filter 15 is disposed at a lower part of the cyclone so that the adhered matter 14 dropped on the filter 15 is vaporized by heat. The vaporized gas is ionized and analyzed by the ion analyzer 8.

The object of the ID authentication is not limited to the ID card. The ID authentication may also be made by way of passport certification, fingerprint authentication, vein authentication or the like. Examples of the usable ion analyzer 8 include a variety of mass spectrometers such as ion trap mass spectrometer, quadrupole mass filter spectrometer, triple quadrupole mass spectrometer, time of flight type mass spectrometer and magnetic sector type mass spectrometer. Alternatively, an ion mobility spectrometer or the like maybe used. Further, the ion mobility spectrometer coupled with the mass spectrometer may also be used. In a case where the mass spectrometer is used as the analyzer, the measured mass spectrum is analyzed so as to identify the components of the adhered matter 14 or to determine the density of the components on the basis of this mass spectrum.

A database storing analysis data of materials is previously created and threshold values for judgement are set. If the density of a detected component exceeds a judgment threshold, then a positive result is given. In that case, whether or not the components are detected may be shown on a display of the device. Otherwise, the device may also be configured not to show the result but to notify the result to a monitoring center in a remote location or to an inspector. In conjunction with the analysis result, a gate for controlling the passage of the subject is opened and closed. Not only in the mass spectrometer but also in the other analyzers such as the ion mobility spectrometer, the adhered matter is analyzed by referring to the database. The gate need not necessarily be unified with the adhered matter inspection device. The gate may be disposed as an independent device. Alternatively, a security agent can also play the same role as the gate.

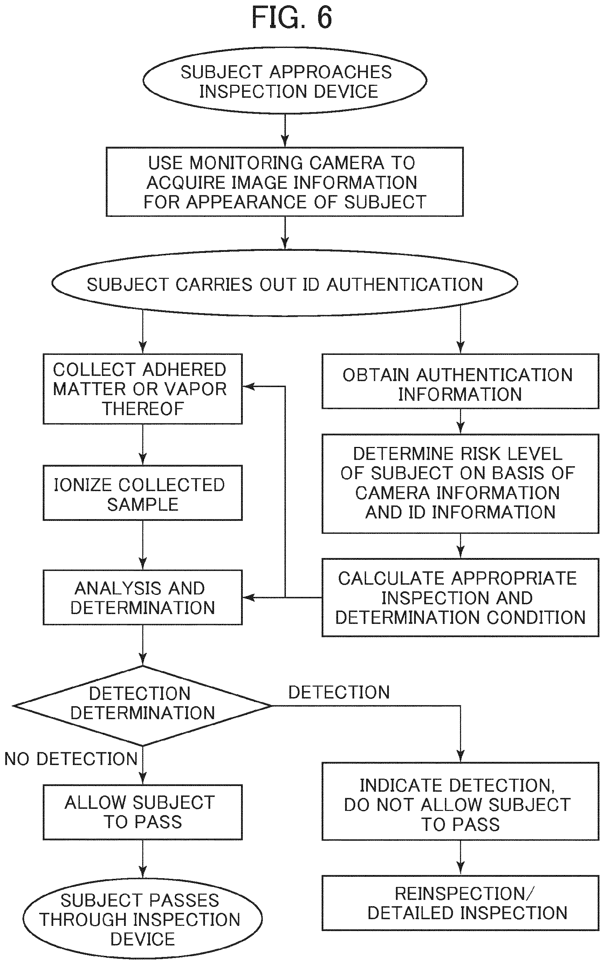

Fig. 6 is a flow chart showing an example of typical inspection procedure. When the subject 2 approaches the gate-type adhered matter inspection device 1, the monitoring camera 3 acquires image information of the appearance of the subject 2. Typical information pieces include the degree of abnormal behavior, baggage size and baggage type. The subject carries out the ID authentication using the ID authentication function of the gate-type adhered matter inspection device 1. At the same time, as shown in Fig. 4 and Fig. 5, the adhered matter on the subject 2 or on the clothes or personal items of the subject or the vapor from the adhered matter is collected. The collected sample is ionized for analysis and judgment.

In parallel with this, the terrorism risk of the subject 2 is calculated by using the ID information and camera information. Typical information pieces for use in the calculation include: degree of abnormal behavior, baggage size, baggage type, criminal record, record of overseas travels, national origin, starting place of overseas travel, destination of overseas travel, flight class (economy, business, etc.), the number of overseas travels, place of employment and the like. It is noted that the calculation of terrorism risk also takes the social circumstances at the time of inspection into consideration. In a period where the number of terrorist attacks in the world increases, the terrorism risk is estimated higher. In the case of a traveler from a country where the terrorist attacks occur frequently, the terrorism risk is estimated higher. The degree of abnormal behavior, the size of baggage carried by the subject and the type of baggage, are acquired from the camera information. According to the embodiment, the information pieces, such as the criminal record, record of overseas travels, national origin, starting place of overseas travel, destination of overseas travel, flight class, number of overseas travels, place of employment, are retrieved from the server 4 based on the ID information. The terrorism risks based on these information pieces are also stored in the server 4 as shown in Fig. 3.

The adhered matter inspection is performed by applying the optimum inspection condition or judgement condition for the inspection of the subject 2 on the basis of the calculated terrorism risk. If the result is negative, the passage is allowed so that the subject can pass through the gate. If the result is positive, on the other hand, the passage is not allowed and the re-inspection or detailed inspection is performed.

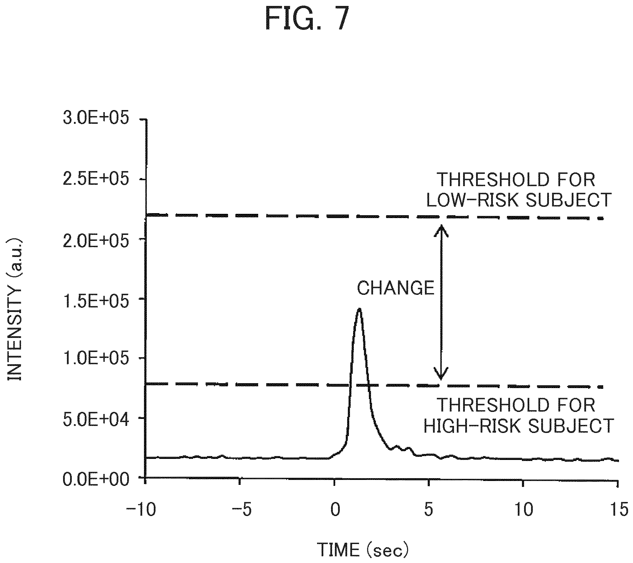

Fig. 7 is a graph showing a typical example of the judgement condition changed on the basis of the terrorism risk. Fig. 7 shows an example of ion chromatogram when a particular explosive is detected by the gate-type adhered matter inspection device 1 employing a mass spectrometer as a sensor, as shown in Fig. 5. In the figure, the abscissa is the time and the ordinate is the signal representing the ion amount of a target material as a detection object. At time zero, the subject 2 carries out the ID authentication while the gas 10 is ejected from the jet nozzle 9. A threshold for judgement is set to a certain value. Time variation of the signal is monitored and analyzed. When the signal exceeds the threshold value, the device gives the positive result. When the positive threshold value is low, the true positive rate rises while the false positive rate rises, too. If the positive threshold value is high, both the true positive rate and the false positive rate decrease. The threshold value is controlled in conformity to the level of terrorism risk of the subject.

While the threshold value may be set on the basis of the absolute value on the ordinate, a different method may also be adopted. A signal value before the start of judgement or a few seconds before time zero is defined as a background value and a standard deviation thereof is calculated. The threshold value is set by the following expression, where 'k' denotes an arbitrary coefficient.

$$\text{Threshold} = \text{background value} + k \times \text{standard deviation}\ldots \quad (2)$$

The background value can vary with the change in the ambient environment of the device. Therefore, the threshold value defined by the expression (2) meets the circumstances more than the threshold defined as the absolute value. The true positive rate can be controlled by varying the coefficient 'k' in conformity to the terrorism risk of the subject. According to the embodiment, therefore, the terrorism risk of the subject is calculated using the information from the monitoring camera or the ID authentication information. The subject with high terrorism risk is judged on the basis of a smaller coefficient 'k' (e.g., k = 3) while the subject with low terrorism risk is judged on the basis of a larger coefficient 'k' (e.g., k=10). In this manner, each subject is inspected under a different judgement condition.

A subject with low terrorism risk has a high threshold value and hence, the false positive rate is lowered so that the probability of undergoing the unwanted re-inspection or detailed inspection decreases. Thus, the inspection throughput is improved. A subject with high terrorism risk is subjected to the inspection under high true positive rate condition whereby the inspection can be performed at high security level. Since the number of the subjects with low terrorism risk is much larger than that of the subjects with high terrorism risk, the system as a whole can achieve a high throughput without lowering the security level. The embodiment can achieve both the high throughput and the high security level. The embodiment does not always require the same coefficient 'k' to be applied to all the target materials. The embodiment permits a small coefficient 'k' to be applied to a material with a high possibility of use in terrorist attacks and a large coefficient 'k' to be applied to a material with a low possibility of use in terrorist attacks. Further, the coefficient may be changed according to the terrorism risk level of each subject.

The information used for the calculation of terrorism risk is not limited only to the monitoring camera information and the ID authentication information. For example, a dangerous material detection device such as a millimeter wave imaging device is usable. The monitoring camera 3 is not limited to that disposed in vicinity of the gate-type adhered matter inspection device 1. Image information from a monitoring camera 3 disposed in a remote location is usable, too. Any device that is capable of terrorism risk calculation by means of a different function from that of the adhered matter inspection device 1 can be employed. While Fig. 1 shows the configuration where the image information from the monitoring camera 3 and the ID authentication information are processed by the server 4, a server function may be incorporated in the camera or the gate-type adhered matter inspection device per se. The closed system in a local environment may sometimes be less susceptible to the influence of network delay so as to be capable of high-speed processing.

Second Embodiment

A typical system configuration according to a second embodiment hereof is the same as that of the first embodiment, as shown in Fig. 1. The embodiment is also the same as the first embodiment in that the terrorism risk of each subject is calculated using the monitoring camera information and the ID authentication information and then, the inspection condition and/or the judgement condition of the adhered matter inspection device 1 are changed.

A plurality of target materials as the inspection object are registered in the database stored in the server 4. In a case where the ion analyzer 8 is a mass spectrometer, an m/z value for which the target material is observed is registered. Typical target materials include but not limited to: military explosives (TNT, PETN, RDX); handmade explosives (TATP, HMTD); combustible liquids (gasoline, kerosene); flammables (sulfur); and the like. The military explosives are hugely destructive, causing heavy damages when used for the purpose of terrorist attack. On the other hand, the explosives are not easily synthesized and are not normally available unless flown out from army. On the other hand, the handmade explosives are easily synthesized from daily necessaries and available to citizens. Therefore, the handmade explosives have high possibility of being used in the terrorist attacks. Citizens can easily purchase the combustible liquids and use them in everyday life. Therefore, it is highly probable that the combustible liquid adheres to the body or personal items of the subject. Sulfur as a combustible material is also used in fireworks and the like. It is highly likely that sulfur is detected as the adhered matter in a case where firework craftworkers or people after the fireworks display finished become subjects.

The true positive rate and the false positive rate are changed depending upon which of the materials is selected as the target. According to the second embodiment, the target material is changed depending upon the terrorism risk level of the subject. The number of target materials is reduced in the inspection of a subject with the low terrorism risk while the number of target materials is increased in the inspection of a subject with the high terrorism risk. In the case of the subject with the low terrorism risk, it is unnecessary to target the combustible liquids and flammables, for example. On the other hand, the handmade explosives are not excluded from the targets because they are likely to be used in the terrorist attacks. Further, in a case where the inspection is performed after the fireworks display finished or in a case where the subject is determined to be a firework craftworker on the basis of the ID authentication information, the procedure is changed to exclude sulfur from the target. In a case where the subject is determined to be a service member with low terrorism risk, military-grade explosives could be excluded from the target. Just as in the first embodiment, the selection of the target materials is also carried out according to the algorithms shown in Fig. 2.

Third Embodiment

A typical system configuration according to a third embodiment hereof is the same as that of the first embodiment, as shown in Fig. 1. The embodiment is also the same as the first embodiment in that the terrorism risk of each subject is calculated using the monitoring camera information and the ID authentication information and then, the inspection condition and/or the judgement condition of the adhered matter inspection device 1 are changed.

In the case where the device as shown in Fig. 5 is used, time from the gas ejection to the detection varies depending upon the material. This is because the steam pressure differs from material to material, making difference in time taken by a heating portion to vaporize the collected adhered matter. Some material can be detected within 1 second after the gas ejection while other material takes 3 seconds or so. For example, materials, such as TNT and TATP, having high steam pressures can be detected at once while a material, such as RDX, having low steam pressure takes a while to be detected. The number of materials to be detected can be varied by changing how many seconds after the gas ejection are to be assigned to the judgement.

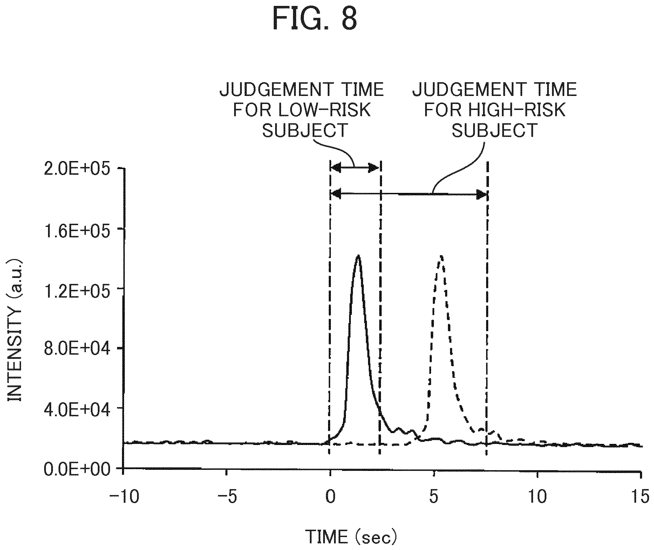

According to the third embodiment, the length of judgement time after the gas ejection is changed depending upon the terrorism risk level of the subject 2. Fig. 8 is a graph showing an exemplary change in the judgement condition. Fig. 8 shows an example of ion chromatogram. When the subject with the low terrorism risk is inspected, the judgement time is shortened to improve the inspection throughput. On the other hand, the judgement time for the subject with the high terrorism risk is increased. As of 2017, the handmade explosive TATP is most likely to be used in the terrorist attacks. As described above, TATP has the high steam pressure and hence, is detectable even in a short judgement time. It is therefore possible to inspect the subject with the low terrorism risk to check whether the adhered matter is TATP or not.

Fourth Embodiment

A typical system configuration according to a fourth embodiment hereof is the same as that of the first embodiment, as shown in Fig. 1. The embodiment is also the same as the first embodiment in that the terrorism risk of each subject is calculated using the monitoring camera information and the ID authentication information and then, the inspection condition and/or the judgement condition of the adhered matter inspection device 1 are changed.

In the ion chromatogram as shown in Fig. 7 and Fig. 8, the calculation is performed with a certain window width defined for the m/z of the target material. TNT, for example, should be detected as a negative ion at m/z = 226.2 on the mass spectrum. Because of the resolution of the mass spectrometer, TNT is detected with a certain degree of m/z margin. When drawing the ion chromatogram, the calculation is performed on the basis of a window width of m/z value of the target material ±0.5.

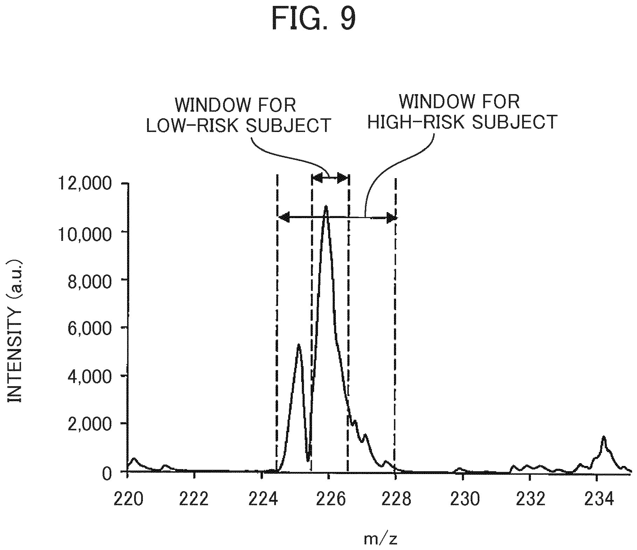

Fig. 9 shows a graph showing an exemplary change in the judgement condition, or a typical mass spectrum. In the case of a narrow window width, an ion chromatograph can be drawn from a peak m/z = 226.2 indicating TNT. With the window width increased, however, adjoining peaks may also affect the data. The peak indicating TNT does not always appear at m/z = 226.2 due to the change in the state of the device or the change in the ambient environment. That is, the fluctuations of the object data occur. If the window width is increased, the influences of the data fluctuations become negligible so that the true positive rate is improved. However, the false positive rate is increased due to the influence of the adjoining peak. It is noted, however, that if the window width is increased too much in a noisy environment, the true positive rate falls because the background value in the expression (2) increases.

According to the fourth embodiment, the m/z window width for ion chromatogram calculation which affects the true positive rate and the false positive rate is changed according to the terrorism risk level of the subject. The m/z window width for the subject with the low terrorism risk is reduced (e.g., $\pm 0.5$) . On the other hand, the m/z window width for the subject with the high terrorism risk is increased (e.g., $\pm 1.5$).

Fifth Embodiment

A typical system configuration according to a fifth embodiment hereof is the same as that of the first embodiment, as shown in Fig. 1. The embodiment is also the same as the first embodiment in that the terrorism risk of each subject is calculated using the monitoring camera information and the ID authentication information and then, the inspection condition and/or the judgement condition of the adhered matter inspection device 1 are changed.

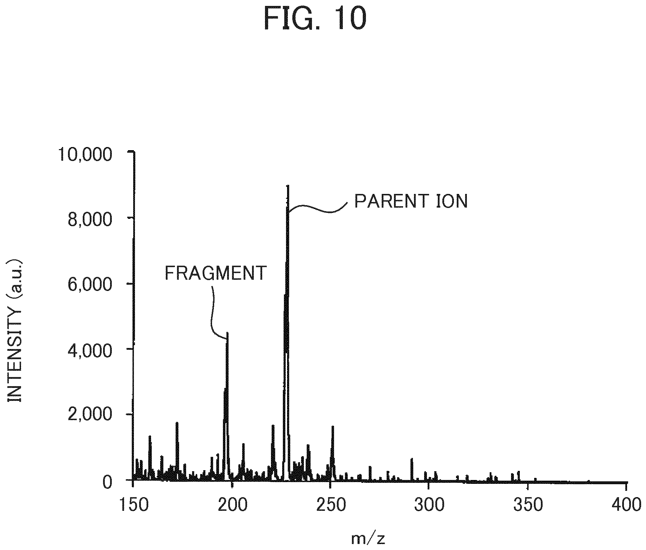

As described above, the adhered matter inspection device 1 holds the database in which a plurality of target materials as the detection object are registered. In a case where the ion analyzer 8 is the mass spectrometer, the m/z values where the target materials are observed are registered. As exemplified by a mass spectrum shown in Fig. 10, the target material is not always detected as peak at one m/z value. In a case where fragment ions or adduct ions with other material are also detected, a plurality of peaks appear in the mass spectrum. In the case of TNT, not only a negative parent ion with one additional electron but also fragment ions may be detected. In the case of RDX, chloride ion adduct ions, lactic acid adduct ions and NOx adduct ions besides the parent ions may be detected.

The false positive rate decreases if plural peaks for one target material are defined as the inspection object and a configuration is made such that positive result is given only when all the peaks are detected (AND judgement) . In the case of the AND judgement, a peak at the lowest sensitivity limits the true positive rate so that the true positive rate decreases in comparison with a case where only a peak at the highest sensitivity is defined as the detection object. On the other hand, both the true positive rate and the false positive rate increase if the positive result is given when any one of the plural detection object peaks is detected (OR judgement).

According to the fifth embodiment, the number of detection objects m/z of the detection target material, and the AND judgement and the OR judgement are changed according to the terrorism risk level of the subject 2. The subject with the low terrorism risk is inspected under conditions of low true positive rate and low false positive rate by making the AND judgement on plural peaks. The subject with the high terrorism risk is inspected under conditions of high true positive rate and high false positive rate by making the OR judgement. Which of the AND judgement and the OR judgement is to be made may be changed depending upon the target material. The condition setting may be made such that, for a subject with extremely high terrorism risk, the OR judgement is made on all the target materials, whereas in the case of a subject with not so high terrorism risk, the OR judgement is made on only TATP having the high probability of being used in the terrorist attack.

Sixth Embodiment

A typical system configuration according to a sixth embodiment hereof is the same as that of the first embodiment, as shown in Fig. 1. The embodiment is also the same as the first embodiment in that the terrorism risk of each subject is calculated using the monitoring camera information and the ID authentication information and then, the inspection condition and/or the judgement condition of the adhered matter inspection device 1 are changed.

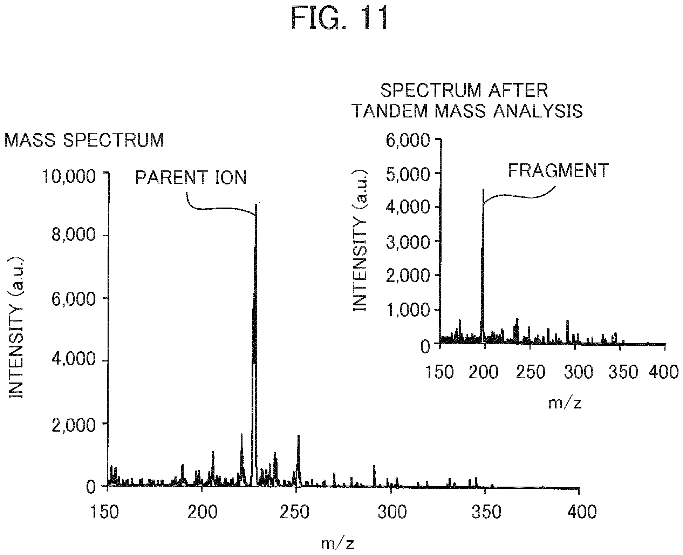

Tandem mass spectrometry is known as a method of raising the true positive rate but decreasing the false positive rate in a case where the mass spectrometer is used as the ion analyzer 8. In the tandem mass spectrometry as shown in Fig. 11, the parent ions detected in the mass spectrum are decomposed by collision-induced dissociation so as to generate fragment ions. When the tandem mass spectrometry is performed, the S/N ratio is normally improved and hence, the true positive rate increases. If materials having the same m/z value are present, the materials can be separated if the fragment ions thereof have different m/z values. Hence, the false positive rate is lowered. However, the inspection time increases.

According to the sixth embodiment, whether or not the tandem mass spectrometry is performed is determined according to the terrorism risk level of the subject 2. As for the subject with the low terrorism risk, the tandem mass spectrometry is not performed so as to end the inspection in short time. As for the subject with the high terrorism risk, on the other hand, the tandem mass spectrometry is performed so as to perform the inspection under high true positive rate condition although the inspection time increases. It is not always necessary to perform the tandem mass spectrometry on all the target materials. A material having a low S/N ratio without the tandem mass spectrometry and a material exhibiting only a single peak on mass spectrum and having a high false positive rate are preferentially selected as a target of the tandem mass spectrometry.

Seventh Embodiment

A typical system configuration according to a seventh embodiment hereof is the same as that of the first embodiment, as shown in Fig. 1. The embodiment is also the same as the first embodiment in that the terrorism risk of each subject is calculated using the monitoring camera information and the ID authentication information and then, the inspection condition and/or the judgement condition of the adhered matter inspection device 1 are changed.

The device shown in Fig. 5 separates and collects the adhered matter by means of gas jet during the ID authentication. The seventh embodiment is configured to change at least one of the number of gas ejections or the gas ejection time according to the terrorism risk of the subject. With the increase in the number of gas ejections or the gas ejection time, the separation efficiency is more improved and the true positive rate increases. However, the inspection time is increased. As for the subject with the low terrorism risk, the inspection is completed in short time by reducing the number of gas ejections and shortening the gas ejection time as exemplified by once and 100ms. As for the subject with the high terrorism risk, on the other hand, the inspection is performed under high true positive rate condition by increasing the number of gas ejections and increasing the gas ejection time, as exemplified by three times and 300ms each. The embodiment differs from the foregoing embodiments in that not the method of analyzing the output data from the sensor but the method of collecting the adhered matter is changed.

Eighth Embodiment

A typical system configuration according to an eighth embodiment hereof is the same as that of the first embodiment, as shown in Fig. 1. The embodiment is also the same as the first embodiment in that the terrorism risk of each subject is calculated using the monitoring camera information and the ID authentication information and then, the inspection condition and/or the judgement condition of the adhered matter inspection device 1 are changed.

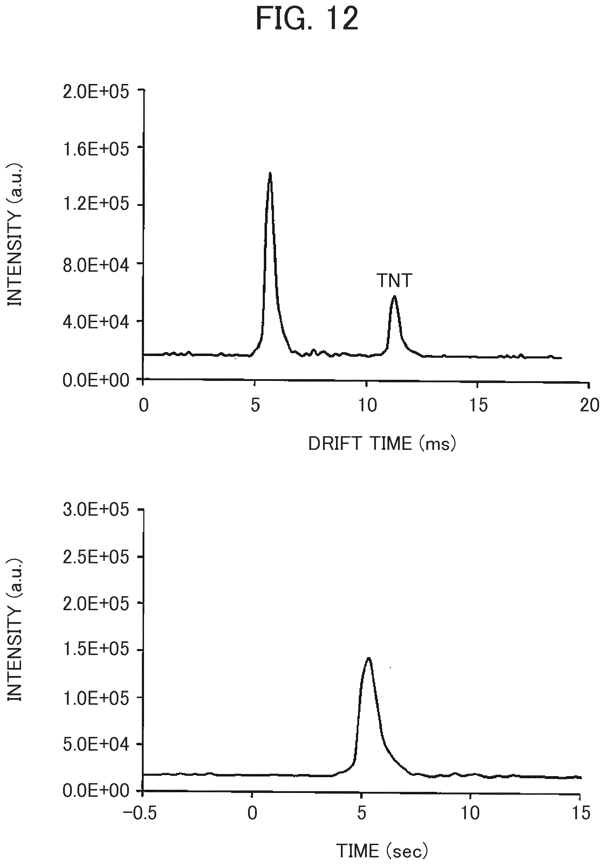

This embodiment employs an ion mobility spectrometer as the ion analyzer 8. Particularly, a typical example is a drift-tube type ion mobility spectrometer. Although the ion mobility spectrometer has lower precisions than the mass spectrometer, the ion mobility spectrometer has an advantage of being less costly. Fig. 12 is a set of graphs showing spectrum obtained by an ion mobility spectrometer and time-varying change in TNT signal. The expression (2) of the first embodiment is applicable to this embodiment, too. While the second embodiment is assumed to employ the mass spectrometer as the ion analyzer 8 and the m/z value of the target material is registered in the database, drift time (ms) of the target material is registered in a case where the ion mobility spectrometer is employed. The change in the judgement time after the gas ejection according to the third embodiment is applicable to the eighth embodiment, too. The change in the window width according to the fourth embodiment is also applicable to the drift time. The change in the number of peaks determined according to the fifth embodiment is applicable to this embodiment, too. The change in the gas ejection condition according to the seventh embodiment is also applicable to this embodiment.

It is noted that the present invention is not limited to the foregoing embodiments and includes a variety of modifications. The foregoing embodiments, for example, are detailed illustrations to clarify the invention. The invention is not necessarily limited to those including all the components described above. Some component of one embodiment can be replaced by some component of another embodiment. Further, some component of one embodiment can be added to the arrangement of another embodiment. A part of the arrangement of each embodiment permits addition of some component of another embodiment, the omission thereof or replacement thereof.

List of Reference Numerals

1:      adhered matter inspection device
2:      subject
3:      monitoring camera
4:      server
8:      ion analyzer
9:      jet nozzle
10:    gas
11:    inspection object
12:    suction fan
13:    cyclone
14:    adhered matter
15:    filter

**Claims**

**1.** An adhered matter inspection method comprising:

a step of acquiring personal information of a subject;

a step of calculating a terrorism risk of the subject by referring the acquired personal information to a database storing a relationship between the personal information and the terrorism risk; and

a step of using an inspection device for collecting an adhered matter on the subject or on an inspection object carried by the subject and/or a vapor from the adhered matter, ionizing and analyzing the collected matter, and determining whether or not the adhered matter is a dangerous material by referring the analysis result to the database; wherein

inspection condition and/or judgement condition of the inspection device is changed on a per-subject basis so that a true positive rate of an inspection is varied according to a level of the calculated terrorism risk.

2. The adhered matter inspection method according to Claim 1, wherein
the inspection condition and/or judgement condition of the inspection device is changed so that at least one of inspection time or false positive rate varies in conjunction with the variation of the true positive rate.

3. The adhered matter inspection method according to Claim 1, wherein
the inspection condition and/or judgement condition of the inspection device is changed so that the true positive rate decreases in the case of a subject with a low calculated terrorism risk and that the true positive rate increases in the case of a subject with a high calculated terrorism risk.

4. The adhered matter inspection method according to Claim 1, wherein
the inspection condition and/or judgement condition of the inspection device is changed so that inspection time decreases in the case of a subject with a low calculated terrorism risk and that the inspection time increases in the case of a subject with a high calculated terrorism risk.

5. The adhered matter inspection method according to Claim 1, wherein
the inspection device separates the adhered matter by ejecting gas to the subject or the inspection object; condenses the separated adhered matter; vaporizes the adhered matter by means of a heating portion and then, ionizes and analyzes the adhered matter.

6. The adhered matter inspection method according to Claim 1, wherein
the analysis is performed by monitoring and analyzing time-varying change in a signal indicating the ion amount of a target material as a detection object, and the true positive rate is varied by changing the method of analyzing the time-varying change.

7. The adhered matter inspection method according to Claim 6, wherein
a value obtained by adding a value obtained by multiplying a standard deviation of a background signal by a coefficient to a value of the background signal before the start of judgement is defined as a judgement threshold value, and the true positive rate is varied by changing the coefficient.

8. The adhered matter inspection method according to Claim 1, wherein
the true positive rate is varied by increasing or decreasing the number of materials selected as the detection objects.

9. The adhered matter inspection method according to Claim 5, wherein
the true positive rate is varied by changing the length of judgment time after a gas ejection.

10. The adhered matter inspection method according to Claim 1, wherein
the true positive rate is varied by changing a m/z window width for calculating ion chromatogram from mass spectrum.

11. The adhered matter inspection method according to Claim 1, wherein
the true positive rate is varied by changing the number of peaks used for judgement, out of the plural peaks of a detection target material observed on mass spectrum.

12. The adhered matter inspection method according to Claim 1, wherein
the true positive rate is varied by changing whether or not to perform collision-induced dissociation.

13. The adhered matter inspection method according to Claim 5, wherein
the true positive rate is varied by changing at least one of the number of gas ejections or gas ejection time.

**14.** The adhered matter inspection method according to Claim 1, wherein
the terrorism risk of the subject is calculated by using at least one of information pieces which are obtained by analyzing monitoring camera information on the subject passing by the inspection device and which include: a degree of abnormal behavior, a size of baggage carried by the subject, or a type of the baggage.

**15.** The adhered matter inspection method according to Claim 1, wherein
the terrorism risk of the subject is calculated by using at least one of information pieces which are acquired by referring ID information of the subject acquired by an ID authentication function annexed to the inspection device to the database and which include: criminal record, record of overseas travels, national origin, starting place of overseas travel, overseas travel destination, flight class, number of overseas travels, or place of employment.

# FIG. 1

CAMERA
INFORMATION

3

ID
INFORMATION

4

PARAMETER
SETTING

2

1

# FIG. 2

# FIG. 3A

| COUNTRY | TERRORISM RISK |
|---------|----------------|
| A | 1 |
| B | 5 |
| C | 10 |

# FIG. 3B

| ESTIMATED BAG WEIGHT (kg) | TERRORISM RISK |
|---------------------------|----------------|
| 0-1 | 1 |
| 1-10 | 5 |
| 10- | 10 |

FIG. 4

FIG. 5

# FIG. 6

SUBJECT APPROACHES
INSPECTION DEVICE

USE MONITORING CAMERA TO
ACQUIRE IMAGE INFORMATION
FOR APPEARANCE OF SUBJECT

SUBJECT CARRIES OUT ID AUTHENTICATION

COLLECT ADHERED
MATTER OR VAPOR
THEREOF

OBTAIN AUTHENTICATION
INFORMATION

IONIZE COLLECTED
SAMPLE

DETERMINE RISK LEVEL
OF SUBJECT ON BASIS OF
CAMERA INFORMATION
AND ID INFORMATION

ANALYSIS AND
DETERMINATION

CALCULATE APPROPRIATE
INSPECTION AND
DETERMINATION CONDITION

DETECTION
DETERMINATION

DETECTION

NO DETECTION

ALLOW SUBJECT
TO PASS

INDICATE DETECTION,
DO NOT ALLOW SUBJECT
TO PASS

SUBJECT PASSES
THROUGH INSPECTION
DEVICE

REINSPECTION/
DETAILED INSPECTION

17

# FIG. 7

# FIG. 8

# FIG. 9

WINDOW FOR
LOW-RISK SUBJECT

WINDOW FOR
HIGH-RISK SUBJECT

# FIG. 10

# FIG. 11

# FIG. 12

**EP 3 644 053 A1**

## INTERNATIONAL SEARCH REPORT

| International application No. |
|---|
| PCT/JP2018/000908 |

A. CLASSIFICATION OF SUBJECT MATTER
Int. Cl. G01N27/62(2006.01)i, G08B25/00(2006.01)i, G08B25/04(2006.01)i

According to International Patent Classification (IPC) or to both national classification and IPC

B. FIELDS SEARCHED

Minimum documentation searched (classification system followed by classification symbols)
Int. Cl. G01N27/62, G08B25/00, G08B25/04

Documentation searched other than minimum documentation to the extent that such documents are included in the fields searched
Published examined utility model applications of Japan       1922-1996
Published unexamined utility model applications of Japan     1971-2018
Registered utility model specifications of Japan             1996-2018
Published registered utility model applications of Japan     1994-2018

Electronic data base consulted during the international search (name of data base and, where practicable, search terms used)
JSTPlus/ JST7580 (JDreamIII)

C. DOCUMENTS CONSIDERED TO BE RELEVANT

| Category* | Citation of document, with indication, where appropriate, of the relevant passages | Relevant to claim No. |
|---|---|---|
| A | WO 2011/024293 A1 (HITACHI, LTD.) 03 March 2011, paragraphs [0019]-[0031], fig. 1, 2 & US 2012/0139736 A1, paragraphs [0055]-[0067], fig. 1, 2 & EP 2472253 A1 | 1-15 |
| A | JP 2004-178258 A (MITSUBISHI HEAVY INDUSTRIES, LTD.) 24 June 2004, paragraphs [0071]-[0076], fig. 3-5 (Family: none) | 1-15 |
| A | US 2009/0034790 A1 (TELESECURITY SCIENCES, INC.) 05 February 2009, paragraphs [0019]-[0022], fig. 1 & WO 2009/017931 A2 | 1-15 |

☒ Further documents are listed in the continuation of Box C.      ☐ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 26.03.2018 | 10.04.2018 |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

24

**INTERNATIONAL SEARCH REPORT**

| International application No. |
| --- |
| PCT/JP2018/000908 |

C (Continuation).    DOCUMENTS CONSIDERED TO BE RELEVANT

| Category* | Citation of document, with indication, where appropriate, of the relevant passages | Relevant to claim No. |
| --- | --- | --- |
| A | WONG, S., BROOKS, N., "Evolving risk-based security: A review of current issues and emerging trends impacting security screening in the aviation industry", Journal of Air Transport Management, September 2015, vol. 48, pp. 60-64, ISSN 0969-6997, in particular section 2. Risk assessment | 1-15 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2011174746 A

- JP 2009080730 A

**Non-patent literature cited in the description**

- *Journal of Air Transport Management,* 2015, vol. 48, 60-64